(19) Europäisches Patentamt
European Patent Office
Office européen des brevets

(11) Veröffentlichungsnummer: **0 124 893**
**A2**

# (12) EUROPÄISCHE PATENTANMELDUNG

(21) Anmeldenummer: **84105052.9**

(22) Anmeldetag: **04.05.84**

(51) Int. Cl.³: **C 07 D 471/04**
A 61 K 31/505, A 61 K 31/515
A 61 K 31/55
//(C07D471/04, 239/00, 221/00),
(C07D471/04, 239/00, 223/00)

(30) Priorität: **05.05.83 CH 2442/83**
**20.03.84 CH 1392/84**

(43) Veröffentlichungstag der Anmeldung:
**14.11.84 Patentblatt 84/46**

(84) Benannte Vertragsstaaten:
**AT BE CH DE FR GB IT LI LU NL SE**

(71) Anmelder: **F. HOFFMANN-LA ROCHE & CO.**
**Aktiengesellschaft**

**CH-4002 Basel(CH)**

(72) Erfinder: **Kaiser, Ado, Dr.**
**Hirsweg 3**
**CH-4415 Lausen(CH)**

(72) Erfinder: **Kienzle, Frank, Dr.**
**Tannwaldweg 9**
**CH-4113 Flüh(CH)**

(74) Vertreter: **Freiherr von Pechmann, Eckehart et al,**
**Patentanwälte Wuesthoff- v. Pechmann-Behrens-Goetz**
**Schweigerstrasse 2**
**D-8000 München 90(DE)**

## (54) Neue Pyrimidonderivate.

(57) Pyrimidonderivate der Formel

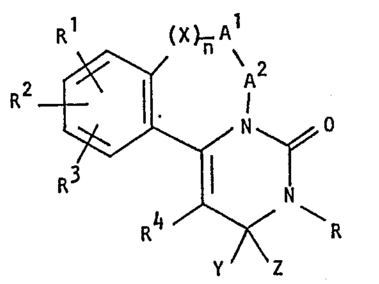

I

worin n die Zahl 1 oder 0,

X, A¹ und A² gegebenenfalls mono- oder di-niederalkyliertes Methylen,

oder X gegebenenfalls niederalkylierter Stickstoff,

R Wasserstoff oder nieder-Alkyl, und Y zusammen mit Z eine Gruppe = $NR^5$,

oder R zusammen mit Z eine N–C–Bindung, und Y eine Gruppe $-N(H)R^5$,

$R^1$, $R^2$ und $R^3$ Wasserstoff, nieder-Alkyl oder nieder-Alkoxy,

oder zwei benachbarte $R^1$, $R^2$ oder $R^3$ Methylendioxy oder Aethylendioxy,

$R^4$ Wasserstoff oder nieder-Alkyl,

$R^5$ gegebenenfalls durch $R^6$, $R^7$ und $R^8$ substituiertes Phenyl,

$R^6$, $R^7$ und $R^8$ Chlor, Fluor, Brom, nieder-Alkyl oder –Alkoxy sind,

wobei falls $R^4$ Wasserstoff und n = 0 ist, mindestens eine der Methylengruppen A¹ und A² mono- oder dineideralkyliert sein soll,

und physiologisch verträgliche Salze davon hemmen die Aggregation der Blutplättchen und sind kreislaufwirksam.

Sie werden ausgehend von entsprechenden chlorierten oder bromierten Pyrimidonderivaten hergestellt.

EP 0 124 893 A2

F.Hoffmann-La Roche & Co.Aktiengesellschaft, Basel/Schweiz 0124893

EP-58 247
(RAN 4044/62)

# Neue Pyrimidonderivate

Die vorliegende Erfindung betrifft neue Pyrimidonderivate, ein Verfahren zu ihrer Herstellung und pharmazeutische Präparate auf der Basis der neuen Verbindungen.

Die erfindungsgemässen Pyrimidonderivate sind Verbindungen der Formel

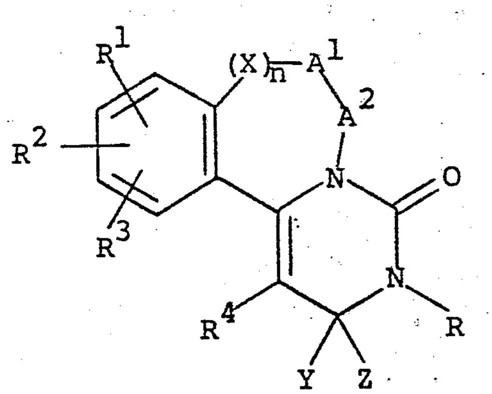

I

worin n die Zahl 1 oder 0,
X, $A^1$ und $A^2$ gegebenenfalls mono- oder di-niederalkyliertes Methylen,
oder X gegebenenfalls niederalkylierter Stickstoff,
R Wasserstoff oder nieder-Alkyl, und Y zusammen mit Z eine Gruppe $=NR^5$,

Mé/7.3.84

oder R zusammen mit Z eine N-C-Bindung, und Y eine Gruppe $-N(H)R^5$,
$R^1$, $R^2$ und $R^3$ Wasserstoff, nieder-Alkyl oder nieder-Alkoxy,
oder zwei benachbarte $R^1$, $R^2$ oder $R^3$ Methylendioxy oder Aethylendioxy,
$R^4$ Wasserstoff oder nieder-Alkyl,
$R^5$ gegebenenfalls durch $R^6$, $R^7$ und $R^8$ substituiertes Phenyl,
$R^6$, $R^7$ und $R^8$ Chlor, Fluor, Brom, nieder-Alkyl oder -Alkoxy sind,
wobei falls $R^4$ Wasserstoff und n = 0 ist, mindestens eine der Methylengruppen $A^1$ und $A^2$ mono- oder di-nieder-alkyliert sein soll,

und physiologisch verträgliche Salze davon.

Der hier verwendete Ausdruck "nieder" bezeichnet geradkettige oder verzweigte Reste mit 1-6, vorzugsweise 1-4, C-Atomen, wie Methyl, Aethyl, Propyl, Isopropyl, n-Butyl und Isobutyl, sowie diesen Alkylresten entsprechende Alkoxyreste. Von Chlor, Fluor und Brom ist ersteres bevorzugt.

Die erfindungsgemässen Pyrimidonderivate können ein oder mehrere asymmetrische Kohlenstoffatome enthalten und können somit als optisch aktive Enantiomere, als Diastereomere oder als Racemate vorliegen. Ferner können die erfindungsgemässen Pyrimidonderivate, insbesondere diejenigen Verbindungen der Formel I und Salze davon, worin R Wasserstoff und Y zusammen mit Z eine Gruppe $=NR^5$, oder worin R zusammen mit Z eine N-C-Bindung und Y eine Gruppe $-N(H)R^5$ sind, in verschiedenen tautomeren Formen vorliegen. Diese Enantiomeren, Diastereomeren, Racemate und Tautomeren sind ebenfalls Gegenstand der Erfindung.

Die Verbindungen der Formel I bilden mit Säuren Salze, die ebenfalls Gegenstand der Erfindung sind. Beispiele solcher Salze sind Salze mit physiologisch verträglichen Mineralsäuren, wie Salzsäure, Bromwasserstoffsäure, Schwefelsäure, Phosphorsäure; oder mit organischen Säuren, wie Methansulfonsäure, Essigsäure, Propionsäure, Zitronensäure, Bernsteinsäure, Aepfelsäure, Fumarsäure, Phenylessigsäure oder Salicylsäure.

Unter den erfindungsgemässen Pyrimidonderivaten sind diejenigen bevorzugt, worin $R^1$ Wasserstoff und $R^2$ und $R^3$ nieder-Alkoxy, insbesondere Methoxy, in para-Stellung zu den angulären C-Atomen des Phenylrings; X, $A^1$ und $A^2$ gegebenenfalls mono- oder dimethyliertes Methylen, oder X gegebenenfalls methylierter Stickstoff; bzw. $R^6$, $R^7$ und $R^8$ Chlor, Brom, nieder-Alkyl oder -Alkoxy sind.

Weiterhin sind Verbindungen der Formel I bevorzugt, worin n 0 oder 1, X Methylen, eines von $A^1$ und $A^2$ Methylen und das andere monomethyliertes Methylen sind, insbesondere solche, worin das C-Atom einer monomethylierten Methylengruppe $A^1$ die R-Konfiguration bzw. das C-Atom einer monomethylierten Methylengruppe $A^2$ die S-Konfiguration hat, und ferner solche, worin R Wasserstoff oder Methyl ist oder R zusammen mit Z eine N-C-Bindung bildet, sowie solche, worin $R^4$ Wasserstoff bzw. $R^5$ Mesityl oder 2,6-Xylyl ist.

Besonders bevorzugt sind die erfindungsgemässen Pyrimidonderivate, worin $R^1$ Wasserstoff und $R^2$ und $R^3$ nieder-Alkoxy, insbesondere Methoxy, in para-Stellung zu den angulären C-Atomen des Phenylrings, n 0 oder 1, X Methylen, eines von $A^1$ und $A^2$ Methylen und das andere monomethyliertes Methylen, wobei das C-Atom einer monomethylierten Methylengruppe $A^1$ die R-Konfiguration bzw. das C-Atom einer monomethylierten Methylengruppe $A^2$ die S-Konfiguration hat, R Wasserstoff oder Methyl ist oder R zusammen mit Z eine N-C-Bindung bildet, $R^4$ Wasserstoff und $R^5$ Mesityl oder 2,6-Xylyl ist, insbesondere die folgenden Verbindungen:

(S)-9,10-Dimethoxy-3,6-dimethyl-2-mesitylimino-2,3,6,7-tetrahydro-4H-pyrimido[6,1-a]isochinolin-4-on,

(S)-6-Aethyl-6,7-dihydro-9,10-dimethoxy-2-mesitylamino-4H-pyrimido[6,1-a]isochinolin-4-on,

(S)-6-Aethyl-9,10-dimethoxy-2-mesitylimino-3-methyl-2,3,6,7-tetrahydro-4H-pyrimido[6,1-a]isochinolin-4-on,

(S)-6-Aethyl-6,7-dihydro-9,10-dimethoxy-2-(2,6-xylidino)-4H-pyrimido[6,1-a]isochinolin-4-on,

(S)-6-Aethyl-9,10-dimethoxy-3-methyl-2,3,6,7-tetrahydro-2-(2,6-xylylimino)-4H-pyrimido[6,1-a]isochinolin-4-on,

6,7-Dihydro-9,10-dimethoxy-2-mesitylamino-6-methyl-4H-pyrimido[6,1-a]isochinolin-4-on,

9,10-Dimethoxy-3,6-dimethyl-2-mesitylimino-2,3,6,7-tetrahydro-4H-pyrimido[6,1-a]isochinolin-4-on,

10,11-Dimethoxy-2-mesitylimino-3-methyl-2,3,7,8-tetrahydro-4H,6H-pyrimido[6,1-a]benzazepin-4-on,

(S)-6,7-Dihydro-9,10-dimethoxy-2-mesitylamino-6-methyl-4H-pyrimido[6,1-a]isochinolin-4-on,

(S)-6,7-Dihydro-9,10-dimethoxy-6-methyl-2-(2,6-xylidino)-4H-pyrimido[6,1-a]isochinolin-4-on,

(S)-9,10-Dimethoxy-3,6-dimethyl-2,3,6,7-tetrahydro-2-(2,6-xylylimino)-4H-pyrimido[6,1-a]isochinolin-4-on und

(R)-9,10-Dimethoxy-3,7-dimethyl-2-mesitylimino-2,3,6,7-tetrahydro-4H-pyrimido[6,1-a]isochinolin-4-on.

Die erfindungsgemässen Verbindungen können dadurch hergestellt werden, dass man

a) eine Verbindung der Formel

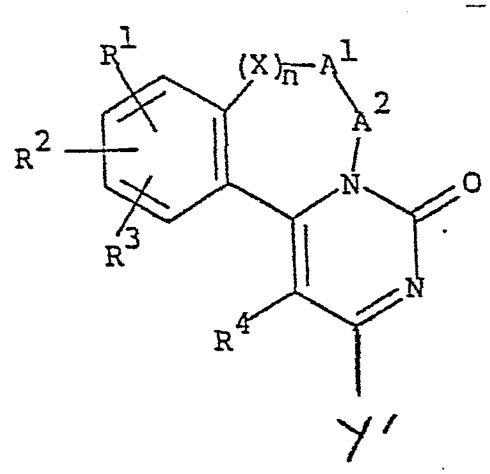

II

worin Y' Chlor oder Brom ist und die restlichen Symbole die oben angegebene Bedeutung haben, mit einem Amin der Formel $R^5NH_2$, worin $R^5$ die oben angegebene Bedeutung hat, umsetzt,

b) gewünschtenfalls eine erhaltene Verbindung der Formel I, worin R Wasserstoff, und Y zusammen mit Z eine Gruppe $=NR^5$ ist, oder worin R zusammen mit Z eine N-C-Bindung bildet und Y eine Gruppe $-N(H)R^5$ ist, und $R^5$ die oben angegebene Bedeutung hat, mit einem nieder-Alkylhalogenid umsetzt,

c) das Produkt der Stufe a) oder b) in Form einer freien Base der Formel I oder eines physiologisch verträglichen Salzes davon isoliert.

Die Verfahrensstufe a) kann in einem Lösungsmittel, vorzugsweise einem aprotischen Lösungsmittel, wie einem halogenierten Kohlenwasserstoff, z.B. Chloroform, zweckmässig in Gegenwart einer Base, wie einem Tri-(nieder-alkyl)-amin, z.B. Triäthylamin, oder einem Alkalimetallcarbonat, z.B. $Na_2CO_3$ oder $K_2CO_3$, bei einer Temperatur bis zu Rückflusstemperatur des Reaktionsgemisches durchgeführt werden. Dabei werden Verbindungen der Formel I erhalten, worin R Wasserstoff, und Y zusammen mit Z eine Gruppe $=NR^5$ oder worin R zusammen mit Z eine N-C-Bindung, und Y eine Gruppe $-N(H)R^5$, und $R^5$ die oben angegebene Bedeutung hat.

Die fakultative Verfahrensstufe b) kann in einem polaren Lösungsmittel, wie einem Keton, z.B. Aceton, oder einem Amid, z.B. Dimethylformamid, bei einer Temperatur bis zu Rückflusstemperatur des Reaktionsgemisches, vorzugsweise bei etwa 100°C, zweckmässig in Gegenwart einer Base, wie eines Alkalimetallcarbonats oder eines gehinderten tertiären Amins, wie eines Tri-(nieder-alkyl)-amins, z.B. Triäthylamin, durchgeführt werden. Dabei werden Verbindungen der Formel I erhalten, worin R nieder-Alkyl, und Y

zusammen mit Z eine Gruppe $=NR^5$ ist, und $R^5$ die oben angegebene Bedeutung hat.

Die Ausgangspyrimidone der Formel II können dadurch hergestellt werden, dass man eine Verbindung der Formel

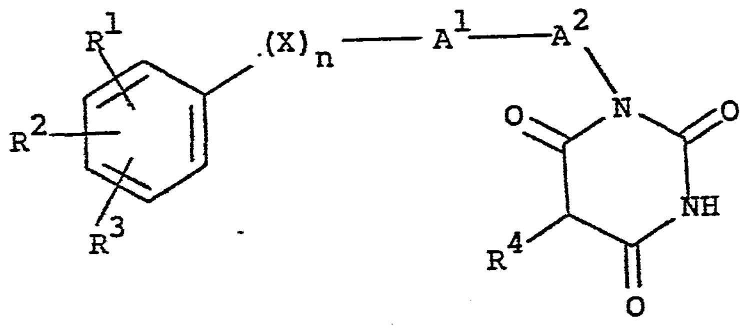

III

worin n, X, $A^1$, $A^2$ und $R^1$-$R^4$ die oben angegebene Bedeutung haben,

mit einem anorganischen Säurechlorid oder -bromid, oder dem entsprechenden anorganischen Chlorid oder Bromid umsetzt.

Diese Reaktion kann mit einem anorganischen Säurechlorid oder -bromid, wie $POCl_3$, $POBr_3$ oder $SOCl_2$, bei einer Temperatur bis zu Rückflusstemperatur des Reaktionsgemisches, oder mit einem dem Säurechlorid oder -bromid entsprechenden anorganischen Chlorid oder Bromid, wie $PCl_5$, bei einer Temperatur bis zu etwa 100°C durchgeführt werden.

Die Barbitursäurederivate der Formel III kann man ausgehend von den entsprechenden Aminen der Formel

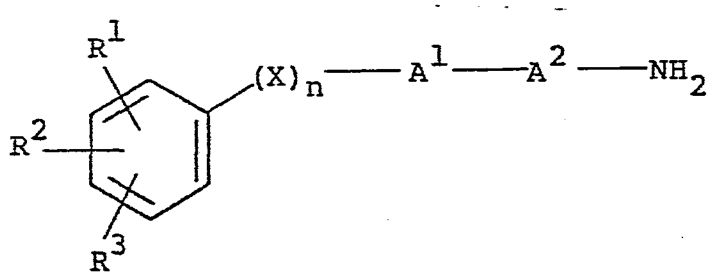

IV

über die Harnstoffderivate der Formel

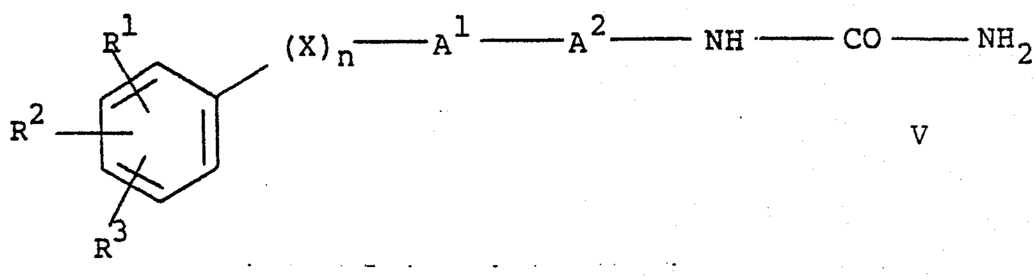

V

worin n, X, $A^1$, $A^2$ und $R^1$-$R^3$ die obige Bedeutung haben,

herstellen. Zweckmässig wird ein Amin IV mit einem Alkaliisocyanat, z.B. Natriumisocyanat, in Anwesenheit einer anorganischen Säure, wie Salzsäure, in einem wässerigen protischen Lösungsmittel, wie einem Alkohol, z.B. Aethanol, umgesetzt. Man kann dann das erhaltene Harnstoffderivat V mit einem Malonsäurederivat der Formel $R^4CH(COOR^6)_2$, worin $R^4$ die obige Bedeutung hat und $R^6$ nieder-Alkyl ist, z.B. mit Malonsäurediäthylester, in einem Alkohol, wie Methanol oder Aethanol, in Anwesenheit eines Alkalimetallalkoholats, z.B. Natriummethanolat oder -äthanolat, umsetzen.

Die Amine der Formel IV sind bekannt oder können in an sich bekannter Weise hergestellt werden, z.B. wie beschrieben in J.Med. Chem. 16, 1973, 480 oder in Tetrahedron 31, 1975, 2595.

Die erfindungsgemässen Pyrimidonderivate können als Heilmittel Verwendung finden. Sie hemmen die Aggregation der Blutplättchen und können daher zur Verhütung von Thrombosen verwendet werden. Ausserdem sind sie kreislaufwirksam, insbesondere antihypertensiv wirksam, und können zur Behandlung bzw. Verhütung von kardiovaskulären Krankheiten Verwendung finden.

Die erfindungsgemässen Pyrimidonderivate können als Heilmittel z.B. in Form pharmazeutischer Präparate Verwendung finden, welche sie oder ihre Salze in Mischung mit einem für die enterale, perkutane oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole oder Vaseline enthalten. Die pharmazeutischen Präparate können in fester Form, z.B. als Tabletten, Dragées, Suppositorien, Kapseln; in halbfester Form, z.B. als Salben; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes oder Puffer. Sie können auch noch andere therapeutisch wertvolle Stoffe enthalten.

Die orale Verabreichung der erfindungsgemässen Pyrimidonderivaten ist bevorzugt. Für den Erwachsenen kommen eine orale Tagesdosis von 0,1 bis 30 mg/kg und eine parenterale Tagesdosis von 0,01 bis 10 mg/kg in Frage, jeweils unter Berücksichtigung der individuellen Bedürfnisse des Patienten und der Verabreichungsform.

Die aggregationshemmende Wirkung wurde nach der Aggregometer-Methode (Nature 194, 1962, 927; 231, 1971, 220) nachgewiesen. Die maximale Aggregationsgeschwindigkeit wurde als Versuchsparameter genommen und die effektive Konzentration ($EC_{50}$) aus Dosis-Wirkungskurven ermittelt. Menschliches plättchenreiches Plasma wurde aus citriertem venösem Blut durch Zentrifugieren erhalten. Die Versuche wurden mit Suspensionen der Testsubstanzen in 0,9% NaCl durchgeführt. 0,18 ml Citratplasma wurden mit 10 µl Suspension der Testverbindungen versetzt und 10 Minuten bei 37°C inkubiert, worauf die Aggregation durch Zusatz von 10 µl einer Suspension von Kollagenfibrillen eingeleitet wurde. Für die Hydrochloride der Produkte von einigen der nachfolgenden Beispiele wurden die nachstehenden $EC_{50}$-Werte (in µM) ermittelt:

| Beispiel: | 2a) | 3) | 4a) | 5a) |
|---|---|---|---|---|
| $EC_{50}$ : | 0,11 | 0,6 | 0,10 | 0,12 |

Das 9,10-Dimethoxy-3,7-dimethyl-2-(o-tolylimino)-2,3,6,7-tetrahydro-4H-pyrimido[6,1-a]isochinolin-4-on-hydrochlorid (das Produkt des Beispiels 6a)2.) hat eine $LD_{50}$ von 2500 mg/kg p.o. an der Maus.

## Beispiel 1

A. Herstellung des Ausgangsmaterials

a) 0,3 Mol 3,4-Dimethoxy-β-methylphenäthylamin werden in 300 ml Aethanol gelöst und mit 0,3 Mol 1N-Salzsäure versetzt. Bei 50°C gibt man dann portionsweise insgesamt 0,31 Mol Natriumisocyanat im Laufe von 90 Minuten zu. Beim Abkühlen kristallisiert das dem Ausgangsamin entsprechende Harnstoffderivat aus. Reiner (3,4-Dimethoxy-β-methylphenäthyl)harnstoff wird abfiltriert und mit kaltem Wasser gewaschen. Smp. 179-180°C.

b) 7,25 g Natrium werden in 420 ml Aethanol aufgelöst. Zu dieser Lösung werden 0,315 Mol Malonsäurediäthylester, gefolgt von 0,3 Mol (3,4-Dimethoxy-β-methylphenäthyl)harnstoff (gelöst in 250 ml Aethanol), gegeben. Das Gemisch wird 20 Stunden unter Rückfluss gekocht, dann abgekühlt und mit 500 ml 1N-Salzsäure versetzt. Die ausfallende 1-(3,4-Dimethoxy-β-methylphenäthyl)barbitursäure wird abfiltriert und mit wässrigem Aethanol gewaschen. Smp. 85-96°C.

c) 0,1 Mol 1-(3,4-Dimethoxy-β-methylphenäthyl)barbitursäure werden in 300 ml Phosphoroxychlorid gelöst und 18 Stunden unter Rückfluss gekocht. Die Lösung wird eingedampft und der Rückstand in einem Gemisch von Eis, Wasser und Chloroform aufgenommen und mit 28%iger Natronlauge auf pH 9 gestellt. Die organische Phase wird abgetrennt; sie enthält 2-Chlor-6,7-dihydro-9,10-dimethoxy-7-methyl-4H-pyrimidino-[6,1-a]isochinolin-4-on.

Eine kleine Portion von dieser organischen Phase wird eingedampft. Der Rückstand wird in Aethylacetat gelöst und an Kieselgel chromatographiert. Nach Umkristallisation aus Aethylacetat-Hexan erhält man das reine Produkt, Smp. 219-220°C.

B. Herstellung des Produktes

Dem grössten Teil der in Stufe A.c) erhaltenen organischen Phase werden 100 ml 2,4,6-Trimethylanilin zugegeben und es wird 20 Stunden unter Rückfluss gekocht. Die Lösung wird dann abgedampft und der Rückstand in Aethylacetat gelöst und an Kieselgel chromatographiert. Reines 6,7-Dihydro-9,10-dimethoxy-2-mesitylamino-7-methyl-4H-pyrimido[6,1-a]isochinolin-4-on wird nach Behandeln mit Salzsäure als Hydrochlorid, Smp. 191-193°C, isoliert (80% Ausbeute).

<u>Beispiel 2</u>

In zu Beispiel 1 analoger Weise wurden hergestellt:

a) ausgehend vom 3,4-Dimethoxy-α-methylphenäthylamin

über den (3,4-Dimethoxy-α-methylphenäthyl)harnstoff, Smp. 172°C

und die (3,4-Dimethoxy-α-methylphenäthyl)barbitursäure, Smp. 162-164°C

das 6,7-Dihydro-9,10-dimethoxy-2-mesitylamino-6-methyl-4H-pyrimido[6,1-a]isochinolin-4-on erhalten; Smp. des Hydrochlorids 199°C;

b) ausgehend vom 3,4-Dimethoxy-β,β-dimethylphenäthylamin

über den (3,4-Dimethoxy-β,β-dimethylphenäthyl)harnstoff, Smp. 152°C

und die 3,4-Dimethoxy-β,β-dimethylphenäthylbarbitursäure, Smp. 172°C

das 6,7-Dihydro-9,10-dimethoxy-2-mesitylamino-7,7-dimethyl-4H-pyrimido[6,1-a]isochinolin-4-on, Smp. des Hydrochlorids 203-205°C.

## Beispiel 3

18 g 6,7-Dihydro-9,10-dimethoxy-2-mesitylamino-7-methyl-4H-pyrimido[6,1-a]isochinolin-4-on werden in 1,5 l Aceton in Anwesenheit von 90 g Kaliumcarbonat mit 270 ml Methyljodid 2 Stunden unter Rückfluss gekocht. Dann wird abfiltriert und das Filtrat wird eingedampft. Der Rückstand wird in Chloroform gelöst und an einer Kieselgelsäule chromatographisch gereinigt. Es werden 9,4 g reines 9,10-Dimethoxy-3,7-dimethyl-2-mesitylimino-2,3,6,7-tetrahydro-4H-pyrimido[6,1-a]isochinolin-4-on, Smp. 156-157°C erhalten. Das entsprechende Hydrochlorid dieser Verbindung schmilzt bei 224°C (Zers.).

## Beispiel 4

In zu Beispiel 3 analoger Weise wurden hergestellt:

a) ausgehend vom 6,7-Dihydro-9,10-dimethoxy-2-mesitylamino-6-methyl-4H-pyrimido[6,1-a]isochinolin-4-on

das 9,10-Dimethoxy-3,6-dimethyl-2-mesitylimino-2,3,6,7-tetrahydro-4H-pyrimido[6,1-a]isochinolin-4-on, Smp. des Hydrochlorids 224°C;

b) ausgehend vom 6,7-Dihydro-9,10-dimethoxy-7,7-dimethyl-2-mesitylamino-4H-pyrimido[6,1-a]isochinolin-4-on

das 9,10-Dimethoxy-2-mesitylimino-2,3,6,7-tetrahydro-3,7,7-trimethyl-4H-pyrimido[6,1-a]isochinolin-4-on, Smp. des Hydrochlorids 192-194°C.

## Beispiel 5

In zu den Beispielen 1 und 3 analoger Weise wurden hergestellt:

a) ausgehend vom 3-(3,4-Dimethoxyphenyl)proylamin

über den [3-(3,4-Dimethoxyphenyl)propyl]harnstoff, Smp. 165-166°C

und die 1-[3-(3,4-Dimethoxyphenyl)propyl]barbitursäure, Smp. 119-121°C

das 10,11-Dimethoxy-2-mesitylimino-3-methyl-2,3,7,8-tetrahydro-4H,6H-pyrimido[6,1-a]benzazepin-4-on erhalten, Smp. des Hydrochlorids 212-214°C;

b) ausgehend vom 3,4-Dimethoxyphenäthylamin

über den 3,4-Dimethoxyphenäthylharnstoff

und die 1-(3,4-Dimethoxyphenäthyl)-5-methylbarbitursäure, Smp. 164-165°C

das 9,10-Dimethoxy-1,3-dimethyl-2-mesitylimino-2,3,6,7-tetrahydro-4H-pyrimido[6,1-a]isochinolin-4-on, Smp. des Hydrochlorid 160°C;

c) ausgehend vom 4-Methoxy-β,β-dimethylphenäthylamin

über den 4-Methoxy-β,β-dimethylphenäthylharnstoff, Smp. 137°C

und die 4-Methoxy-β,β-dimethylphenäthylbarbitursäure, Smp. 175°C

das 2-Mesitylimino-10-methoxy-2,3,6,7-tetrahydro-3,7,7-trimethyl-4H-pyrimido[6,1-a]isochinolin-4-on, Smp. des Hydrochlorids 216-217°C.

Beispiel 6

In zu den Beispielen 1 und 3 analoger Weise wurden hergestellt:

a)1. das 6,7-Dihydro-9,10-dimethoxy-7-methyl-2-(o-toluidino)-4H-pyrimido[6,1-a]isochinolin-4-on und

2. das 9,10-Dimethoxy-3,7-dimethyl-2-(o-tolylimino)-2,3,6,7-tetrahydro-4H-pyrimido[6,1-a]isochinolin-4-on, Smp. des Hydrochlorids 192-193°C;

b)1. das 6,7-Dihydro-9,10-dimethoxy-7-methyl-2-(2,6-xylidino)-4H-pyrimido[6,1-a]isochinolin-4-on und

2. das 9,10-Dimethoxy-3,7-dimethyl-2,3,6,7-tetrahydro-(2,6-xylylimino)-4H-pyrimido[6,1-a]isochinolin-4-on, Smp. des Hydrochlorids 241-242°C;

c)1. das 6,7-Dihydro-9,10-dimethoxy-2-(3,5-dimethoxyanilino) 7-methyl-4H-pyrimido[6,1-a]isochinolin-4-on und

2. das 2,3,6,7-Tetrahydro-9,10-dimethoxy-3,7-dimethyl-2-(3,5-dimethoxyphenylimino)-4H-pyrimido[6,1-a]isochinolin-4-on, Smp. des Hydrochlorids 226°C;

d)1. das 6,7-Dihydro-9,10-dimethoxy-7-methyl-2-(3,4,5-trimethoxyanilino)-4H-pyrimido[6,1-a]isochinolin-4-on und

2. das 9,10-Dimethoxy-3,7-dimethyl-2,3,6,7-tetrahydro-2-(3,4,5-trimethoxyphenylimino)-4H-pyrimido[6,1-a]isochinolin-4-on, Smp. des Hydrochlorids 256-257°C;

e)1. das 6,7-Dihydro-9,10-dimethoxy-2-(p-fluoranilino)-7-methyl-4H-pyrimido[6,1-a]isochinolin-4-on und

2. das 2,3,6,7-Tetrahydro-9,10-dimethoxy-3,7-dimethyl-2-(p-fluorphenylimino)-4H-pyrimido[6,1-a]isochinolin-4-on,

Smp. des Hydrochlorids 240°C;

f)1. das 6,7-Dihydro-9,10-dimethoxy-7-methyl-2-(2,4-xylidino)-4H-pyrimido[6,1-a]isochinolin-4-on und

2. das 9,10-Dimethoxy-3,7-dimethyl-2,3,6,7-tetrahydro-2-(2,4-xylylimino)-4H-pyrimido[6,1-a]isochinolin-4-on, Smp. des Hydrochlorids 230-231°C;

g)1. das 2-Anilino-6,7-dihydro-9,10-dimethoxy-7-methyl-4H-pyrimido[6,1-a]isochinolin-4-on und

2. das 9,10-Dimethoxy-3,7-dimethyl-2-phenylimino-2,3,6,7-tetrahydro-4H-pyrimido[6,1-a]isochinolin-4-on, Smp. des Hydrochlorids 232-233°C;

h)1. das 6,7-Dihydro-9,10-dimethoxy-2-(p-methoxyanilino)-7-methyl-4H-pyrimido[6,1-a]isochinolin-4-on und

2. das 9,10-Dimethoxy-3,7-dimethyl-2-(p-methoxyphenylimino)-2,3,6,7-tetrahydro-4H-pyrimido[6,1-a]isochinolin--on, Smp. des Hydrochlorids 215°C;

i)1. das 6,7-Dihydro-9,10-dimethoxy-2-(m-fluoranilino)-7-methyl-4H-pyrimido[6,1-a]isochinolin-4-on und

2. das 9,10-Dimethoxy-3,7-dimethyl-2-(m-fluorphenylimino)-2,3,6,7-tetrahydro-4H-pyrimido[6,1-a]isochinolin-4-on, Smp. des Hydrochlorids 228-230°C;

j)1. das 2-(6-Chlor-2-toluidino)-6,7-dihydro-9,10-dimethoxy-7-methyl-4H-pyrimido[6,1-a]isochinolin-4-on und

2. das 2-(6-Chlor-2-tolylimino)-9,10-dimethoxy-3,7-dimethyl-2,3,6,7-tetrahydro-4H-pyrimido[6,1-a]isochinolin-4-on, Smp. des Hydrochlorids 211-212°C;

k)1. das 2-(2,6-Diäthylanilino)-6,7-dihydro-9,10-dimethoxy-7-methyl-4H-pyrimido[6,1-a]isochinolin-4-on und

2. das 2-(2,6-Diäthylphenylimino)-9,10-dimethoxy-3,7-dimethyl-2,3,6,7-tetrahydro-4H-pyrimido[6,1-a]isochinolin-4-on, Smp. des Hydrochlorids 196-197°C;

l)1. das 6,7-Dihydro-2-(2,6-diisopropylanilino)-9,10-dimethoxy-7-methyl-4H-pyrimido[6,1-a]isochinolin-4-on und

2. das 2-(2,6-Diisopropylphenylimino)-9,10-dimethoxy-3,7-dimethyl-2,3,6,7-tetrahydro-4H-pyrimido[6,1-a]isochinolin-4-on, Smp. des Hydrochlorids 233°C.

## Beispiel 7

In zu den Beispielen 1 und 3 analoger Weise wurden folgende Verbindungen hergestellt:

a) ausgehend vom α-Aethyl-3,4-dimethoxyphenäthylamin, Smp. des Hydrochlorids 150-152°C,

1. das 6-Aethyl-6,7-dihydro-9,10-dimethoxy-2-mesitylamino-4H-pyrimido[6,1-a]isochinolin-4-on, Smp. des Hydrochlorids 219°C, und

2. das 6-Aethyl-9,10-dimethoxy-2-mesitylimino-3-methyl-2,3,6,7-tetrahydro-4H-pyrimido[6,1-a]isochinolin-4-on, Smp. des Hydrochlorids 207-209°C;

b) ausgehend von 2-Amino-3-(3,4-dimethoxyphenyl)butan,

1. das 6,7-Dihydro-9,10-dimethoxy-6,7-dimethyl-2-mesitylamino-4H-pyrimido[6,1-a]isochinolin-4-on und

2. das 9,10-Dimethoxy-2-mesitylimino-2,3,6,7-tetrahydro-3,6,7-trimethyl-4H-pyrimido[6,1-a]isochinolin-4-on, Smp. des Hydrochlorids 236-237°C;

c) ausgehend von 3,4-Diäthoxy-α-methylphenäthylamin, Smp. des Hydrochlorids 144-146°C;

1. das 9,10-Diäthoxy-6,7-dihydro-2-mesitylamino-6-methyl-4H-pyrimido[6,1-a]isochinolin-4-on, Smp. des Hydrochlorids 208-210°C, und

2. das 9,10-Diäthoxy-3,6-dimethyl-2-mesitylimino-2,3,6,7-tetrahydro-4H-pyrimido[6,1-a]isochinolin-4-on, Smp. des Hydrochlorids 119-121°C,

d) ausgehend vom 2,3-Dimethoxy-α-methylphenäthylamin,

das 8,9-Dimethoxy-3,6-dimethyl-2-mesitylimino-2,3,6,7-tetrahydro-4H-pyrimido[6,1-a]isochinolin-4-on, Smp. des Hydrochlorids 220°C;

e) ausgehend vom α-Methyl-3,4,5-trimethoxyphenäthylamin

das 3,6-Dimethyl-2-mesitylimino-2,3,6,7-tetrahydro-9,10,11-trimethoxy-4H-pyrimido[6,1-a]isochinolin-4-on, Smp. des Hydrochlorids 221°C;

f) ausgehend vom α-Methyl-3,4-methylendioxyphenäthylharnstoff

das 3,6-Dimethyl-2-mesitylimino-9,10-methylendioxy-2,3,6,7-tetrahydro-4H-pyrimido[6,1-a]isochinolin-4-on, Smp. des Hydrochlorids 300°C;

g) ausgehend vom 3-(3,4-Dimethoxyphenyl)butylamin über den 3-(3,4-Dimethoxyphenyl)harnstoff, Smp. des Hydrochlorids 87-89°C,

das 10,11-Dimethoxy-3,8-dimethyl-2-mesitylimino-2,3,7,8-tetrahydro-4H,6H-pyrimido[6,1-a]benzazepin-4-on, Smp. des Hydrochlorids 234-235°C;

h) ausgehend vom (S)-3,4-Dimethoxy-α-methylphenäthylamin über die 1-[(S)-3,4-Dimethoxy-α-methylphenäthyl]barbitursäure, Smp. 178-179°C, $[\alpha]_D$ +93,6° (c = 1%, Methanol),

1. das (S)-6,7-Dihydro-9,10-dimethoxy-2-mesitylamino-6-methyl-4H-pyrimido[6,1-a]isochinolin-4-on, Smp. des Hydrochlorids 196°C, $[\alpha]_D$ +43,3° (c = 2%, Methanol), und

2. das (S)-9,10-Dimethoxy-3,6-dimethyl-2-mesitylimino-2,3,6,7-tetrahydro-4H-pyrimido[6,1-a]isochinolin-4-on,Smp. des Hydrochlorids 196°C, $[\alpha]_D$ -13° (c = 2%, Wasser);

i) ausgehend vom (R)-3,4-Dimethoxy-α-methylphenäthylamin über die 1-[(R)-3,4-Dimethoxy-α-methylphenäthyl]barbitursäure, Smp. 177-178°C, $[\alpha]_D$ -92,1° ( c = 1%, Methanol),

1. das (R)-6,7-Dihydro-9,10-dimethoxy-2-mesitylamino-6-methyl-4H-pyrimido[6,1-a]isochinolin-4-on,Smp. des Hydrochlorids 196°C, $[\alpha]_D$ -39.6° (c = 2%, Methanol), und

2. das (R)-9,10-Dimethoxy-3,6-dimethyl-2-mesitylimino-2,3,6,7-tetrahydro-4H-pyrimido[6,1-a]isochinolin-4-on, Smp. des Hydrochlorids 195-196°C, $[\alpha]_D$ +13° (c = 2,5%, Wasser);

j) ausgehend von der 1-[(S)-3,4-Dimethoxy-α-methylphenäthyl]barbitursäure

1. das (S)-6,7-Dihydro-9,10-dimethoxy-6-methyl-2-(2,6-xylidino)-4H-pyrimido[6,1-a]isochinolin-4-on, Smp. des Hydrochlorids 182-183°C; $[\alpha]_D$ +45,6° (c = 0,5, Methanol), und

2. das (S)-9,10-Dimethoxy-3,6-dimethyl-2,3,6,7-tetrahydro-2-(2,6-xylylimino)-4H-pyrimido[6,1-a]isochinolin-4-on, Smp. des Hydrochlorids 167-168°C,$[\alpha]_D$ +30,1° (c = 2%, Methanol);

k) ausgehend vom (S)-3,4-Dimethoxy-β-methylphenäthylamin über den [(S)-3,4-Dimethoxy-β-methylphenäthyl]harnstoff, Smp. 186-187°C, $[\alpha]_D$ -41° (c = 1%, Methanol),

das (R)-9,10-Dimethoxy-3,7-dimethyl-2-mesitylimino-2,3,6,7-tetrahydro-4H-pyrimido[6,1-a]isochinolin-4-on, Smp. des Hydrochlorids 219-221°C, $[\alpha]_D$ -18,0° (c = 2%, Methanol);

l) ausgehend vom (R)-3,4-Dimethoxy-β-methylphenäthylamin über den [(R)-3,4-Dimethoxy-β-methylphenäthyl]harnstoff, Smp. 187-188°C, $[\alpha]_D$ +45° (c = 1%, Methanol),

das (S)-9,10-Dimethoxy-3,7-dimethyl-2-mesitylimino-2,3,6,7-tetrahydro-4H-pyrimido[6,1-a]isochinolin-4-on, Smp. des Hydrochlorids 220-221°,$[\alpha]_D$ +13° (c = 2%, Methanol),

m) ausgehend vom (S)-α-Aethyl-3,4-dimethoxyphenäthylamin über den (S)-α-Aethyl-3,4-dimethoxyphenäthylharnstoff, Smp. 135-137°C, $[\alpha]_D$ +11,4 (c = 1%, Methanol),

1. das (S)-6-Aethyl-6,7-dihydro-9,10-dimethoxy-2-mesitylamino-4H-pyrimido[6,1-a]isochinolin-4-on, Smp. des Hydrochlorids 189-193°C, $[\alpha]_D$ +62,5° (c = 2%, Methanol),

2. das (S)-6-Aethyl-9,10-dimethoxy-2-mesitylimino-3-methyl-2,3,6,7-tetrahydro-4H-pyrimido[6,1-a]isochinolin-4-on, Smp. des Hydrochlorids 166-170°C, $[\alpha]_D$ +47,4° (c = 2%, Methanol),

3. das (S)-6-Aethyl-6,7-dihydro-9,10-dimethoxy-2-(2,6-xylidino)-4H-pyrimido[6,1-a]isochinolin-4-on, Smp. des Hydrochlorids 171-175°C, $[\alpha]_D$ +58,0° (c = 2%, Methanol), und

4. das (S)-6-Aethyl-9,10-dimethoxy-3-methyl-2,3,6,7-tetrahydro-2-(2,6-xylylimino)-4H-pyrimido[6,1-a]isochinolin-4-on, Smp. des Hydrochlorids 148-152°C, $[\alpha]_D$ +49,4° (c = 2%, Methanol).

Beispiel 8

In Analogie zu Beispiel 3 wird unter Verwendung von 1-Propyljodid an Stelle von Methyljodid das 9,10-Dimethoxy-2-mesitylimino-7-methyl-3-propyl-2,3,6,7-tetrahydro-4H-pyrimido[6,1-a]isochinolin-4-on hergestellt, Smp. des Hydrochlorids 152-154°C.

## Beispiel A

In üblicher Weise werden Tabletten folgender Zusammensetzung hergestellt:

| | |
|---|---|
| Das Hydrochlorid eines Pyrimidonderivates der Formel I | 185,0 mg |
| Milchzucker | 15,0 mg |
| Maisstärke | 37,5 mg |
| Wasserlösliches Polyvinylpyrrolidon | 10,0 mg |
| Magnesiumstearat | 2,5 mg |
| Gesamtgewicht pro Tablette | 250,0 mg |

## Beispiel B

In üblicher Weise werden Gelatinesteckkapseln folgender Zusammensetzung hergestellt:

| | |
|---|---|
| Das Hydrochlorid eines Pyrimidonderivates der Formel I | 200,0 mg |
| Wasserlösliches Polyvinylpyrrolidon | 2,0 mg |
| Maisstärke | 43,0 mg |
| Talk | 4,5 mg |
| Magnesiumstearat | 0,5 mg |
| Gesamtgewicht pro Kapsel | 250,0 mg |

## Beispiel C

In üblicher Weise wird eine Injektionslösung folgender Zusammensetzung hergestellt:

| | |
|---|---|
| Das Hydrochlorid eines Pyrimidonderivates der Formel I | 115,0 mg |
| Glycerinformal | 2,4 ml |
| Wasser | 4,0 ml |

## Patentansprüche

1. Pyrimidonderivate der Formel

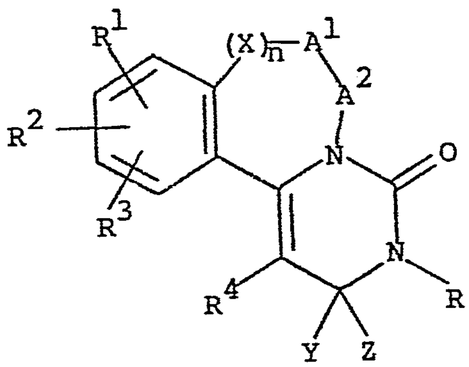

I

worin n die Zahl 1 oder 0, X, $A^1$ und $A^2$ gegebenenfalls mono- oder di-niederalkyliertes Methylen, oder X gegebenenfalls niederalkylierter Stickstoff, R Wasserstoff oder nieder-Alkyl, und Y zusammen mit Z eine Gruppe $=NR^5$, oder R zusammen mit Z eine N-C-Bindung, und Y eine Gruppe $-N(H)R^5$, $R^1$, $R^2$ und $R^3$ Wasserstoff, nieder-Alkyl oder nieder-Alkoxy, oder zwei benachbarte $R^1$, $R^2$ oder $R^3$ Methylendioxy oder Aethylendioxy, $R^4$ Wasserstoff oder nieder-Alkyl, $R^5$ gegebenenfalls durch $R^6$, $R^7$ und $R^8$ substituiertes Phenyl, $R^6$, $R^7$ und $R^8$ Chlor, Fluor, Brom, nieder-Alkyl oder -Alkoxy sind, wobei falls $R^4$ Wasserstoff und n = 0 ist, mindestens eine der Methylengruppen $A^1$ und $A^2$ mono- oder di-nieder-alkyliert sein soll, und physiologisch verträgliche Salze davon.

2. Verbindungen nach Anspruch 1, worin X, $A^1$ und $A^2$ gegebenenfalls mono- oder dimethyliertes Methylen, oder X gegebenenfalls methylierter Stickstoff, und $R^6$, $R^7$ und $R^8$ Chlor, Brom, nieder-Alkyl oder -Alkoxy sind.

3. Verbindungen nach Anspruch 1 oder 2, worin $R^1$ Wasserstoff und $R^2$ und $R^3$ nieder-Alkoxy, insbesondere Methoxy, in para-Stellung zu den angulären C-Atomen des Phenylrings sind.

4. Verbindungen nach Anspruch 1, 2 oder 3, worin n 0 oder 1, X Methylen, eines von $A^1$ und $A^2$ Methylen und das andere monomethyliertes Methylen ist.

5. Verbindungen nach Anspruch 4, worin das C-Atom einer monomethylierten Methylengruppe $A^1$ die R-Konfiguration bzw. das C-Atom einer monomethylierten Methylengruppe $A^2$ die S-Konfiguration hat.

6. Verbindungen nach einem der Ansprüche 1-5, worin R Wasserstoff oder Methyl ist oder R zusammen mit Z eine N-C-Bindung bildet.

7. Verbindungen nach einem der Ansprüche 1-6, worin $R^4$ Wasserstoff ist.

8. Verbindungen nach einem der Ansprüche 1-7, worin $R^5$ Mesityl oder 2,6-Xylyl ist.

9. Verbindungen nach einem der Ansprüche 1-8, worin $R^1$ Wasserstoff und $R^2$ und $R^3$ nieder-Alkoxy, insbesondere Methoxy, in para-Stellung zu den angulären C-Atomen des Phenylrings, n 0 oder 1, X Methylen, eines von $A^1$ und $A^2$ Methylen und das andere monomethyliertes Methylen ist, wobei das C-Atom einer monomethylierten Methylengruppe $A^1$ die R-Konfiguration bzw. das C-Atom einer monomethylierten Methylengruppe $A^2$ die S-Konfiguration hat, R Wasserstoff oder Methyl oder Methyl ist oder R zusammen mit Z eine N-C-Bindung bildet, $R^4$ Wasserstoff und $R^5$ Mesityl oder 2,6-Xylyl ist.

10. Eine Verbindung aus der Gruppe der folgenden:

(S)-9,10-Dimethoxy-3,6-dimethyl-2-mesitylimino-2,3,6,7-tetrahydro-4H-pyrimido[6,1-a]isochinolin-4-on,

(S)-6-Aethyl-6,7-dihydro-9,10-dimethoxy-2-mesitylamino-4H-pyrimido[6,1-a]isochinolin-4-on,

(S)-6-Aethyl-9,10-dimethoxy-2-mesitylimino-3-methyl-2,3,6,7-tetrahydro-4H-pyrimido[6,1-a]isochinolin-4-on,

(S)-6-Aethyl-6,7-dihydro-9,10-dimethoxy-2-(2,6-xylidino)-4H-pyrimido[6,1-a]isochinolin-4-on und

(S)-6-Aethyl-9,10-dimethoxy-3-methyl-2,3,6,7-tetrahydro-2-(2,6-xylylimino)-4H-pyrimido[6,1-a]isochinolin-4-on.

11. Eine Verbindung aus der Gruppe der folgenden:

6,7-Dihydro-9,10-dimethoxy-2-mesitylamino-6-methyl-4H-pyrimido[6,1-a]isochinolin-4-on,

9,10-Dimethoxy-3,6-dimethyl-2-mesitylimino-2,3,6,7-tetrahydro-4H-pyrimido[6,1-a]isochinolin-4-on,

10,11-Dimethoxy-2-mesitylimino-3-methyl-2,3,7,8-tetrahydro-4H,6H-pyrimido[6,1-a]benzazepin-4-on,

(S)-6,7-Dihydro-9,10-dimethoxy-2-mesitylamino-6-methyl-4H-pyrimido[6,1-a]isochinolin-4-on,

(S)-6,7-Dihydro-9,10-dimethoxy-6-methyl-2-(2,6-xylidino)-4H-pyrimido[6,1-a]isochinolin-4-on,

(S)-9,10-Dimethoxy-3,6-dimethyl-2,3,6,7-tetrahydro-2-(2,6-xylylimino)-4H-pyrimido[6,1-a]isochinolin-4-on und

(R)-9,10-Dimethoxy-3,7-dimethyl-2-mesitylimino-2,3,6,7-tetrahydro-4H-pyrimido[6,1-a]isochinolin-4-on.

12. Verbindungen nach einem der Ansprüche 1-11 zur Anwendung als pharmazeutische Mittel.

13. Verbindungen nach einem der Ansprüche 1-11 zur Anwendung als Mittel zur Hemmung der Blutplättchenaggregation.

14. Verbindungen nach einem der Ansprüche 1-11 zur Anwendung als kreislaufwirksame Mittel.

15. Pharmazeutisches Präparat , gekennzeichnet durch einen Gehalt an einer Verbindung nach einem der Ansprüche 1-11.

16. Pharmazeutisches Präparat nach Anspruch 15 zur Hemmung der Blutplättchenaggregation.

17. Kreislaufwirksames Präparat nach Anspruch 15.

18. Verfahren zur Herstellung der Verbindungen nach Anspruch 1, dadurch gekennzeichnet, dass man

a) eine Verbindung der Formel

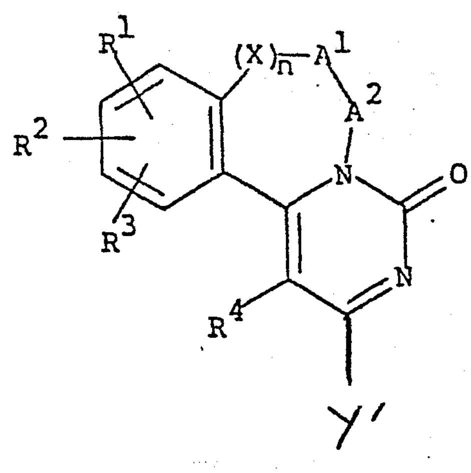

II

worin Y' Chlor oder Brom und die restlichen Symbole die in Anspruch 1 angegebene Bedeutung haben, mit einem Amin der Formel $R^5NH_2$, worin $R^5$ die in Anspruch 1 angegebene Bedeutung hat, umsetzt,

b) gewünschtenfalls eine erhaltene Verbindung der Formel I, worin R Wasserstoff, und Y zusammen mit Z eine Gruppe $=NR^5$ ist, oder worin R zusammen mit Z eine N-C-Bindung bildet und Y eine Gruppe $-N(H)R^5$ ist, und $R^5$ die in Anspruch 1 angegebene Bedeutung hat, mit einem nieder-Alkylhalogenid umsetzt,

c) das Produkt der Stufe a) oder b) in Form einer freien Base der Formel I oder eines physiologisch verträglichen Salzes davon isoliert.

***

Patentansprüche für Oesterreich

1. Verfahren zur Herstellung von Pyrimidonderivaten der Formel

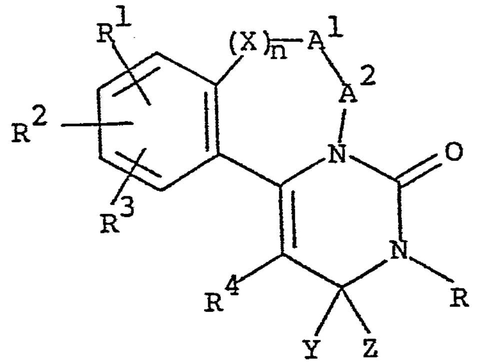

I

worin n die Zahl 1 oder 0,
X, $A^1$ und $A^2$ gegebenenfalls mono- oder di-niederalkyliertes Methylen,
oder X gegebenenfalls niederalkylierter Stickstoff,
R Wasserstoff oder nieder-Alkyl, und Y zusammen mit Z eine Gruppe $=NR^5$,
oder R zusammen mit Z eine N-C-Bindung, und Y eine Gruppe $-N(H)R^5$,
$R^1$, $R^2$ und $R^3$ Wasserstoff, nieder-Alkyl oder nieder-Alkoxy,
oder zwei benachbarte $R^1$, $R^2$ oder $R^3$ Methylendioxy oder Aethylendioxy,
$R^4$ Wasserstoff oder nieder-Alkyl,
$R^5$ gegebenenfalls durch $R^6$, $R^7$ und $R^8$ substituiertes Phenyl,
$R^6$, $R^7$ und $R^8$ Chlor, Fluor, Brom, nieder-Alkyl oder -Alkoxy sind,
wobei falls $R^4$ Wasserstoff und n = 0 ist, mindestens eine der Methylengruppen $A^1$ und $A^2$ mono- oder di-niederalkyliert sein soll,

und von physiologisch verträglichen Salzen davon, dadurch gekennzeichnet, dass man

a) eine Verbindung der Formel

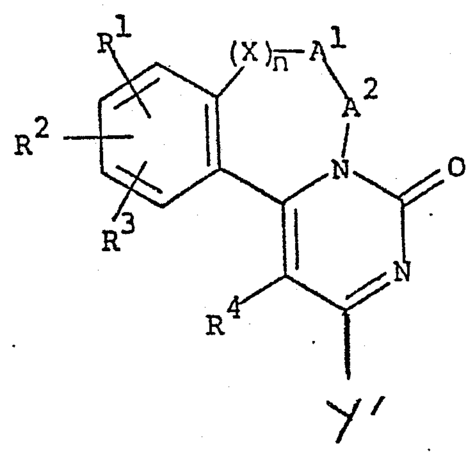

II

worin Y' Chlor oder Brom ist und die restlichen Symbole die oben angegebene Bedeutung haben,

mit einem Amin der Formel $R^5NH_2$, worin $R^5$ die oben angegebene Bedeutung hat, umsetzt,

b) gewünschtenfalls eine erhaltene Verbindung der Formel I, worin R Wasserstoff, und Y zusammen mit Z eine Gruppe $=NR^5$ ist, oder worin R zusammen mit Z eine N-C-Bindung bildet, und Y eine Gruppe $-N(H)R^5$ ist, und $R^5$ die oben angegebene Bedeutung hat, mit einem nieder-Alkylhalogenid umsetzt,

c) das Produkt der Stufe a) oder b) in Form einer freien Base der Formel I oder eines physiologisch verträglichen Salzes davon isoliert.

2. Verfahren nach Anspruch 1 zur Herstellung von Verbindungen nach Anspruch 1, worin X, $A^1$ und $A^2$ gegebenenfalls mono- oder dimethyliertes Methylen, oder X gegebenenfalls methylierter Stickstoff, und $R^6$, $R^7$ und $R^8$ Chlor, Brom, nieder-Alkyl oder -Alkoxy sind, dadurch gekennzeichnet, dass man entsprechend substituierte Ausgangsmaterialien einsetzt.

3. Verfahren nach Anspruch 1 oder 2 zur Herstellung von Verbindungen nach Anspruch 1 oder 2, worin $R^1$ Wasserstoff und $R^2$ und $R^3$ nieder-Alkoxy, insbesondere Methoxy, in para-Stellung zu den angulären C-Atomen des Phenylrings sind, dadurch gekennzeichnet, dass man entsprechend substituierte Ausgangsmaterialien einsetzt.

4. Verfahren nach Anspruch 1, 2 oder 3 zur Herstellung von Verbindungen nach Anspruch 1, 2 oder 3, worin n 0 oder 1, X Methylen, eines von $A^1$ und $A^2$ Methylen und das andere monomethyliertes Methylen sind, dadurch gekennzeichnet, dass man entsprechend substituierte Ausgangsmaterialien einsetzt.

5. Verfahren nach Anspruch 4 zur Herstellung von Verbindungen nach Anspruch 4, worin das C-Atom einer monomethylierten Methylengruppe $A^1$ die R-Konfiguration bzw. das C-Atom einer monomethylierten Methylengruppe $A^2$ die S-Konfiguration hat, dadurch gekennzeichnet, dass man entsprechend substituierte Ausgangsmaterialien einsetzt.

6. Verfahren nach einem der Ansprüche 1-5 zur Herstellung von Verbindungen nach einem der Ansprüche 1-5, worin R Wasserstoff oder Methyl ist oder R zusammen mit Z eine N-C-Bindung bildet, dadurch gekennzeichnet, dass man entsprechend substituierte Ausgangsmaterialien einsetzt.

7. Verfahren nach einem der Ansprüche 1-6 zur Herstellung von Verbindungen nach einem der Ansprüche 1-6, worin $R^4$ Wasserstoff ist, dadurch gekennzeichnet, dass man entsprechend substituierte Ausgangsmaterialien einsetzt.

8. Verfahren nach einem der Ansprüche 1-7 zur Herstellung von Verbindungen nach einem der Ansprüche 1-7, worin $R^5$ Mesityl oder 2,6-Xylyl ist, dadurch gekennzeichnet, dass man entsprechend substituierte Ausgangsmaterialien einsetzt.

9. Verfahren nach einem der Ansprüche 1-8 zur Herstellung von Verbindungen nach einem der Ansprüche 1-8, worin $R^1$ Wasserstoff und $R^2$ und $R^3$ nieder-Alkoxy, insbesondere Methoxy, in para-Stellung zu den angulären C-Atomen des Phenylrings, n 0 oder 1, X Methylen, eines von $A^1$ und $A^2$ Methylen und das andere monomethyliertes Methylen ist, wobei das C-Atom einer monomethylierten Methylengruppe $A^1$

die R-Konfiguration bzw. das C-Atom einer monomethylierten Methylengruppe $A^2$ die S-Konfiguration hat, R Wasserstoff oder Methyl ist oder R zusammen mit Z eine N-C-Bindung bildet, $R^4$ Wasserstoff und $R^5$ Mesityl oder 2,6-Xylyl ist, dadurch gekennzeichnet, dass man entsprechend substituierte Ausgangsmaterialien einsetzt.

10. Verfahren nach Anspruch 1 zur Herstellung einer Verbindung aus der Gruppe der folgenden:

(S)-9,10-Dimethoxy-3,6-dimethyl-2-mesitylimino-2,3,6,7-tetrahydro-4H-pyrimido[6,1-a]isochinolin-4-on,
(S)-6-Aethyl-6,7-dihydro-9,10-dimethoxy-2-mesitylamino-4H-pyrimido[6,1-a]isochinolin-4-on,
(S)-6-Aethyl-9,10-dimethoxy-2-mesitylimino-3-methyl-2,3,6,7-tetrahydro-4H-pyrimido[6,1-a]isochinolin-4-on,
(S)-6-Aethyl-6,7-dihydro-9,10-dimethoxy-2-(2,6-xylidino)-4H-pyrimido[6,1-a]isochinolin-4-on und
(S)-6-Aethyl-9,10-dimethoxy-3-methyl-2,3,6,7-tetrahydro-2-(2,6-xylylimino)-4H-pyrimido[6,1-a]isochinolin-4-on,

dadurch gekennzeichnet, dass man entsprechend substituierte Ausgangsmaterialien einsetzt.

11. Verfahren nach Anspruch 1 zur Herstellung einer Verbindung aus der Gruppe der folgenden:

6,7-Dihydro-9,10-dimethoxy-2-mesitylimino-6-methyl-4H-pyrimido[6,1-a]isochinolin-4-on,
9,10-Dimethoxy-3,6-dimethyl-2-mesitylamino-2,3,6,7-tetrahydro-4H-pyrimido[6,1-a]isochinolin-4-on,
10,11-Dimethoxy-2-mesitylimino-3-methyl-2,3,7,8-tetrahydro-4H,6H-pyrimido[6,1-a]benzazepin-4-on,
(S)-6,7-Dihydro-9,10-dimethoxy-2-mesitylamino-6-methyl-4H-pyrimido[6,1-a]isochinolin-4-on,
(S)-6,7-Dihydro-9,10-dimethoxy-6-methyl-2-(2,6-xylidino)-4H-pyrimido[6,1-a]isochinolin-4-on,

(S)-9,10-Dimethoxy-3,6-dimethyl-2,3,6,7-tetrahydro-2-(2,6-xylylimino)-4H-pyrimido[6,1-a]isochinolin-4-on und

(R)-9,10-Dimethoxy-3,7-dimethyl-2-mesitylimino-2,3,6,7-tetrahydro-4H-pyrimido[6,1-a]isochinolin-4-on,

dadurch gekennzeichnet, dass man entsprechend substituierte Ausgangsmaterialien einsetzt.